# EUROPÄISCHE PATENTSCHRIFT

(11) **EP 0 809 482 B1**
(45) Veröffentlichungstag und Bekanntmachung des Hinweises auf die Patenterteilung: **09.10.2002**
(21) Anmeldenummer: 96903982.5
(22) Anmeldetag: 05.02.1996
(51) Int. Cl.: A61K 7/11, A61K 7/13

(54) **MITTEL MIT KORROSIVEN BESTANDTEILEN UND ABGABEVORRICHTUNG**
AGENT CONTAINING CORROSIVE COMPONENTS AND DISPENSER
PRODUIT COMPORTANT DES COMPOSANTS CORROSIFS ET DISPOSITIF DISTRIBUTEUR

(30) Priorität: 13.02.1995 DE 19504502
(43) Veröffentlichungstag der Anmeldung: 03.12.1997
(73) Patentinhaber: Henkel Kommanditgesellschaft auf Aktien, 40191 Düsseldorf (DE)
(72) Erfinder: OBERKOBUSCH, Doris, D-40591 Düsseldorf (DE); HOLLENBERG, Detlef, D-40699 Erkrath (DE); MEHL, Dietlof, D-40477 Düsseldorf (DE); SCHNEIDER, Hans, D-41366 Schwalmtal (DE)
(86) Internationale Anmeldenummer: EP9600466
(87) Internationale Veröffentlichungsnummer: WO96025138

(56) Entgegenhaltungen:
- GB-A- 763 035
- US-A- 3 644 210
- US-A- 4 776 500
- US-A- 5 348 731

## Beschreibung

Die Erfindung betrifft ein Mittel mit korrosiven Komponenten und eine Abgabevorrichtung für dieses Mittel.

Die Zahl der Produkte, die als Sprays appliziert werden, hat in der Vergangenheit ständig zugenommen. Sowohl die Formulierung neuer Produkte als auch die Anpassung bestehender Produkte an veränderte Randbedingungen haben zu ständig neuen Innovationen auf diesem Gebiet geführt. Als Beispiele seien nur der Ersatz der Fluorchlorkohlenwasserstoffe aufgrund der Ozondiskussion sowie die Entwicklung sogenannter Pumpsprays, bei denen im Prinzip auf ein spezielles Treibmittel verzichtet wird, genannt. Bezüglich weiterer Informationen sei hier ausdrücklich auf den Artikel in Soap, Perfumery, Cosmetics (1994), S.33-35, verwiesen. Problematisch war und blieb aber bisher die Applikation von Mitteln, die stark korrosive Inhaltsstoffe enthalten, in Sprayform. Hier gab es bisher keine Lösungen, um die Korrosionsprobleme innerhalb der Abgabevorrichtung in den Griff zu bekommen. So treten in der Regel bereits nach so kurzer Lagerzeit erhebliche Korrosionsprobleme an Teilen wie dem Ventil und den Innenflächen von Metall-Spraydosen auf, daß Konsumentenprodukte aufgrund ihrer üblichen Lager- und Nutzungsdauern für eine solche Konfektionierung nicht in Frage kommen.

Ein wichtiges Einsatzgebiet für Sprays war und ist der Bereich der Körperpflege. Ausgehend von Deosprays, Haarsprays und Rasierschäumen wurden immer mehr Produkte als Aerosol konfektioniert. Dabei standen eine Vielzahl von Ursachen, wie beispielsweise die Möglichkeit der gleichmäßigen Applikation, das Gefühl und die Wünsche der Verbraucher Pate. So werden zwischenzeitlich so unterschiedliche Produkte wir temporäre Färbemittel, Haarkuren und Haarfestiger entsprechend den Verbraucherwünschen als Aerosole auf das Haar aufgebracht.

Es gibt jedoch nach wie vor Produktgruppen, die aufgrund von unverzichtbaren Inhaltsstoffen als ungeeignet für die Aerosol-Formulierungen gelten, bzw. bei Aerosolformulierungen Nachteile oder unerwünschte Nebeneffekte aufweisen. Eine solche Gruppe stellen Formulierungen mit größeren Mengen an Wasserstoffperoxid dar. Diese sind in der Regel so korrosiv, daß selbst bei lackierten Spraydosen unweigerlich Korrosionseffekte in der Dose auftreten. Weitere Probleme ergeben sich bei der Verwendung der marktüblichen Ventile. Trotz intensiver Bemühungen war es nicht möglich, befriedigende Produkte zu entwickeln. Zwar war es durch spezielle Vorkehrungen möglich, diese Nachteile kurzfristig zu unterdrücken, doch stellten diese keine Lösungen für den Bereich der Konsumentenprodukte dar, bei denen von der Herstellung bis zur Verwendung der letzten Produktreste mitunter mehrere Jahre vergehen können. Hier muß für den Kunden sichergestellt sein, daß Produkt und Applikationssystem keiner Veränderung wie Korrosion unterliegen und auch nach dieser Zeit noch einwandfrei funktionieren.

Es wurde nun überraschenderweise gefunden, daß je nach vorliegendem System, bei Beachtung bestimmter Punkte diese Probleme überwunden werden können. Von entscheidender Bedeutung ist dabei die Wahl der verwendeten Materialien für die Ventile der Abgabevorrichtung. Auf diese Weise können nicht nur spezielle Körperpflegeprodukte sondern auch Produkte auf einer Vielzahl anderer Gebiete nunmehr als Sprays formuliert werden.

Gegenstand der Erfindung ist daher ein korrosive Komponenten enthaltendes Mittel zum Ausbringen aus einer Abgabevorrichtung mittels eines Treibmittels, dadurch gekennzeichnet, daß das Mittel einen Gehalt an korrosiven Bestandteilen von 0,1-50 Gew.-%, bezogen auf das gesamte Mittel, aufweist und mit dem Mittel in Kontakt kommende Teile des Ventils der Abgabevorrichtung aus Edelstahl bestehen oder mit einem Polyolefin beschichtet sind.

Die Art der erfindungsgemäßen Mittel und deren Verwendungszweck unterliegen keinen prinzipiellen Beschränkungen. In einer bevorzugten Ausführungsform der vorliegenden Erfindung handelt es sich jedoch um ein kosmetisches Mittel. Besonders bevorzugte Mittel sind Fixiermittel, die im Rahmen einer Dauerwellbehandlung oder einer oxidativen Färbebehandlung verwendet werden.

Ebenfalls praktisch keinen Beschränkungen unterliegt die Art des korrosiven Mittels. Korrosive Mittel, für die die erfindungsgemäße Lehre besonders geeignet ist, sind Zubereitungen mit einem Gehalt an Wasserstoffperoxid, Alkalimetallhydroxiden und Phosphonsäuren. Ganz besonders geeignet ist die erfindungsgemäße Lehre für Mittel mit einem Gehalt an Wasserstoffperoxid, wie er in bestimmten Haarbehandlungsmitteln und anderen Produkten üblich ist. Solche Wasserstoffperoxidgehalte liegen üblicherweise oberhalb von ca. 2 Gew.-% und unterhalb von ca. 12 Gew.-%, jeweils bezogen auf das gesamte Mittel.

Weiterhin hat es sich in vielen Fällen als vorteilhaft erwiesen, wenn die korrosiven Mittel nur geringe Mengen an Halogeniden aufweisen. Dies kann insbesondere dann gegeben sein, wenn die korrosive Komponente des Mittels Wasserstoffperoxid ist. Insbesondere kann es dann von Vorteil sein, wenn der Gehalt an Halogeniden weniger als 0,5 Gew.-%, berechnet als Natriumchlorid und bezogen auf das gesamte Mittel, beträgt. Bei bestimmten Zubereitungen kann es sogar bevorzugt sein, den Halogenidgehalt auf 0,1 Gew.-% oder gar auf 0,01 Gew.-%, entsprechend der oben genannten Definition, zu beschränken.

Die Aussagen zu den Halogeniden sind vor allem bedeutsam hinsichtlich der Chloride; bei diesen sollten die genannten Bedingungen möglichst exakt eingehalten werden. Bei Fluoriden, Bromiden und insbesondere Iodiden können die genannten Grenzen in einzelnen Fällen weniger kritisch sein. Dennoch wird der Fachmann sich auch bei diesen Halogenidtypen in der Regel an die genannten Obergrenzen halten.

Prinzipiell ist es nicht von entscheidender Bedeutung, ob es sich um anorganische oder organische Halogenide handelt. Unter anorganischen Halogeniden werden erfindungsgemäß Salze der Halogenwasserstoffsäuren mit anorganischen Basen verstanden. Bei den Kationen dieser anorganischen Basen kann es sich somit beispielsweise um Natrium, Kalium, Lithium, Magnesium, Ammonium und Aluminium handeln.

Organische Halogenide sind beispielsweise kationische Tenside vom Typ der Dialkyldimethylammoniumhalogenide oder Alkyltrimethylammoniumhalogenide.

Etwas weniger kritisch sind die Bedingungen, wenn die Halogenidionen als Gegenionen für polymere Verbindungen in das Mittel eingebracht werden. So unterliegen die entsprechenden Polymeren, die in den erfindungsgemäßen Mitteln in den meisten Fällen ohnehin nur in untergeordneten Mengen eingesetzt werden, in den erfindungsgemäßen Mitteln in vielen Fällen keinen Beschränkungen im Vergleich zu den bekannten Mitteln. Stehen jedoch für den gleichen Anwendungszweck sowohl halogenidhaltige als auch halogenidfreie Polymeren mit vergleichbarem Leistungsspektrum zur Verfügung, so wird der Fachmann in der Regel die halogenidfreien Produkte bevorzugen.

Weniger kritisch ist auch der Gehalt anderer Salze, wie Sulfate, Carbonate, Nitrate, Citrate, Lactate und Salicylate, in dem erfindungsgemäßen Mittel. In der Regel wirken sich übliche Gehalte an solchen Salzen, seien sie nun organischer oder anorganischer Natur, nicht negativ aus. Trotzdem kann es in bestimmten Fällen vorteilhaft sein, insbesondere auf weitere anorganische Salze zu verzichten.

In einer besonders bevorzugten Ausführungsform werden den erfindungsgemäßen Mitteln keine Halogenide zugesetzt. Der Gehalt der erfindungsgemäßen Mitteln an solchen Salzen ist dann auf die Mengen begrenzt, die herstellungsbedingt oder aufgrund der jeweiligen Zubereitung in den für die erfindungsgemäßen Mittel verwendeten Rohstoffen enthalten sind. Hier kann der Fachmann durch Wahl besonders salz/halogenid-armer oder salz/halogenidfreier Rohstoffe steuernd eingreifen.

Bei den im Rahmen der erfindungsgemäßen Lehre verwendeten Abgabevorrichtung handelt es sich üblicherweise um Spray- oder Pumpspray-Dosen.

Auch im Kosmetikbereich werden für die erfindungsgemäßen Mittel im wesentlichen diese zwei Arten von Abgabevorrichtungen eingesetzt. Ist das Treibmittel Teil der Zubereitung, beispielsweise ein Kohlenwasserstoff oder ein komprimiertes oder verflüssigtes Gas, so daß das ganze System unter einem permanenten Überdruck steht, so wird als Abgabevorrichtung üblicherweise eine, gegebenenfalls lackierte, Blechdose verwendet. Ist das Produkt dagegen als sogenanntes Pumpspray konfektioniert, bei dem direkt vor der Applikation durch Pumpvorgänge kurzfristig ein geringer Überdruck aufgebaut wird, so wird als Abgabevorrichtung üblicherweise eine, häufig durchsichtige oder durchscheinende, Kunststoffdose verwendet.

Während der zweite Fall hinsichtlich der korrosiven Bestandteile keine weiteren Probleme aufwirft, sind die Dinge bei Verwendung von Blechdosen wesentlich komplizierter. Bei korrosiven Inhaltsstoffen werden die verwendeten Blechdosen üblicherweise innen mit einer Lackschicht versehen. Dies reicht in einer Vielzahl von Fällen bereits aus, um eine Korrosion der Dosen-Innenseite zu verhindern. Bei besonders korrosiven Mitteln ist es allerdings möglich, die Blechdose zusätzlich noch mit einem Innenbeutel aus einem inerten, beweglichen Kunststoffmaterial auszustatten. Dieser Innenbeutel wird üblicherweise aus einem Polyolefin, insbesondere einem Polyethylen, bevorzugt niederer Dichte, hergestellt. Aber auch Weich-PVC und Latex können als Material für den Innenbeutel verwendet werden.

Gemäß einer besonderen Ausführungsform der vorliegenden Verbindung wird eine Spraydose mit einem Innenbeutel aus Polyethylen verwendet.

Ein entscheidender Parameter der erfindungsgemäßen Lehre betrifft das die Abgabevorrichtung verschließende Ventil.

Für Abgabevorrichtungen im Bereich der Kosmetik kommen insbesondere sogenannte Tellerventile in Betracht. Wesentliche Bestandteile dieser Ventile sind Steigrohr, Gehäuse, Teller, Tellerdichtung, Kegel und Kegeldichtung. Dem oben genannten Artikel aus Soap, Perfumery, Cosmetics, auf den ausdrücklich Bezug genommen wird, kann das allgemeine Bauprinzip solcher im Kosmetikbereich verwendeter Ventile entnommen werden.

Es hat sich als zwingende Voraussetzung erwiesen, daß zumindest der Ventilteller und/oder die Ventilfeder aus bestimmten Materialien bestehen.

Gemäß einer bevorzugten Ausführungsform der Erfindung besteht der Ventilteller und/oder die Ventilfeder aus Edelstahl. Bevorzugte Edelstahlqualitäten sind V2A und insbesondere V4A.

Gemäß einer weiteren bevorzugten Ausführungsform der Erfindung werden übliche Ventilteller und/oder Ventilfedern verwendet, die mit einer Polyolefinschicht, insbesondere einer Polyethylenschicht, überzogen sind.

Weiterhin kann es vorteilhaft sein, eine Kegeldichtung aus Nitrilkautschuk, insbesondere der Qualität Buna 418, wie sie von der Firma Perfect-Valois Ventil GmbH vertrieben wird, zu verwenden.

Wie bereits oben ausgeführt, sind erfindungsgemäß bevorzugte Mittel Fixiermittel, die im Rahmen einer Dauerwellbehandlung verwendet werden, sowie Mittel zur oxidativen Färbebehandlung.

Das übliche Verfahren zur Dauerwell-Behandlung menschlicher Haare, das sowohl zur Erzeugung von Locken und Wellen in glattem Haar als auch zur Glättung von gekräuselten Haaren angewendet wird, funktioniert wie folgt:

Das menschliche Haar, eine Keratinfaser, wird mechanisch verformt und die Verformung durch geeignete Hilfsmittel festgelegt. Vor und/oder nach dieser Verformung behandelt man die Faser mit der wäßrigen Zubereitung einer keratinreduzierenden Substanz und spült nach einer Einwirkungszeit mit Wasser oder einer wäßrigen Lösung. In einem zweiten Schritt behandelt man dann die Faser mit der wäßrigen Zubereitung eines Oxidationsmittels. Nach einer Einwirkungszeit wird auch dieses ausgespült und die Faser von den mechanischen Verformungshilfsmitteln (Wickler, Papilloten) befreit.

Die wäßrige Zubereitung des Keratinreduktionsmittels ist üblicherweise alkalisch eingestellt, damit die Faser quillt und auf diese Weise ein tiefes Eindringen der keratinreduzierenden Substanz in die Faser ermöglicht wird. Die keratinreduzierende Substanz spaltet einen Teil der Disulfid-Bindungen des Keratins zu -SH-Gruppen, so daß es zu einer Lockerung der Peptidvernetzung und infolge der Spannung der Faser durch die mechanische Verformung zu einer Neuorientierung des Keratingefüges kommt. Unter dem Einfluß des Oxidationsmittels werden erneut Disulfid-Bindungen geknüpft, und auf diese Weise wird das Keratingefüge in der vorgegebenen Verformung neu fixiert.

Im weiteren werden folgende Bezeichnungen verwendet:
- "Wellmittel" für die wäßrige Zubereitungen der keratinreduzierenden Substanz und
- "Fixiermittel" für die wäßrige Zubereitung des Oxidationsmittels.

Als Reduktionsmittel werden heute fast ausschließlich Thioglycolsäure oder deren Salze oder Ester verwendet; weiterhin sind Verbindungen wie beispielsweise Thiomilchsäure, Thioäpfelsäure, Mercaptoethansulfonsäure sowie deren Salze und Ester, Cysteamin, Cystein, Bunte Salze und Alkalisalze der schwefligen Säure als Reduktionsmittel verwendbar. Die entsprechenden Zubereitungen sind neutral bis alkalisch, d. h. bei einem pH-Wert von ca. 7 - 9,5, eingestellt.

Die Wellmittel können als gebrauchsfertige Mischungen z.B. in Creme-, Geloder Aerosolform sowie als Flüssigkeit formuliert werden, die vom Friseur oder Endverbraucher direkt angewendet werden können. Es hat sich in manchen Fällen aber als vorteilhaft oder notwendig erwiesen, wenn die Mittel als sogenannte 2-Komponenten-Mischungen formuliert werden, die erst vom Anwender zum gebrauchsfertigen Wellmittel vermischt werden. In diesem Fall enthält eine Formulierung das Reduktionsmittel in einem geeigneten Träger, z.B. Wasser oder einer Emulsion. Weiterhin können die Wellmittel alle für Wellmittel bekannten Inhaltsstoffe enthalten; in diesem Zusammenhang wird ausdrücklich auf die dem Fachmann bekannten Monographien, beispielsweise von Schrader (Grundlagen und Rezepturen der Kosmetik) und Janistyn (Handbuch der Kosmetika und Riechstoffe) verwiesen.

Beliebige Vertreter dieser Wellmittel können mit den erfindungsgemäßen Fixiermitteln kombiniert werden, wobei in einer bevorzugten Ausführungsform sowohl Wellmittel als auch Fixiermittel als Aerosol formuliert werden.

Zwingender Bestandteil der erfindungsgemäßen Fixiermittel ist Wasserstoffperoxid. Der pH-Wert solcher wäßriger H₂O₂-Zubereitungen, die üblicherweise etwa 0,5 bis 3,0 Gew.-% H₂O₂ enthalten, liegt bevorzugt bei 2 bis 5; er wird durch anorganische Säuren, bevorzugt Phosphorsäure, eingestellt. Gleichfalls geeignet sind Fixiermittel auf enzymatischer Basis (Peroxidasen), die geringere Mengen an H₂O₂ enthalten.

Es kann auch bevorzugt sein, die erfindungsgemäßen Fixiermittel als 2-Komponenten-System zu formulieren. Die beiden Komponenten, von denen die eine bevorzugt eine Wasserstoffperoxidlösung und die andere die übrigen Bestandteile enthält, werden ebenfalls erst kurz vor der Anwendung vermischt.

Weiterhin können die erfindungsgemäßen Fixiermittel alle für Fixiermittel im Rahmen einer Dauerwellbehandlung üblichen Bestandteile enthalten.

Bevorzugte weitere Komponenten sind kationische, zwitterionische und amphotere Tenside.

Beispiele für die in den erfindungsgemäßen Mitteln verwendbaren kationischen Tenside sind insbesondere spezielle, halogenidfreie quartäre Ammoniumverbindungen. Bevorzugt sind beispielsweise Alkyloxyethylammoniumphosphat, das unter der Bezeichnung Dehyquart^{R} SP im Handel ist.

Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{R} vertriebenen Dialkylammoniummethosulfate und Methyl-hydroxyalkyl-dialkoyloxyalkyl-ammoniummethosulfate.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beipielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter amphoteren Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Weitere bevorzugte Komponenten der erfindungsgemäßen Zubereitungen sind kationische, zwitterionische oder amphotere Polymere.

Die erfindungsgemäß verwendbaren kationischen Polymeren enthalten innerhalb des Polymergerüstes kationische Gruppen. Diese Gruppen können Teil der Polymerkette sein; sie können sich aber auch in Seitenketten befinden, die über Zwischenglieder mit einer Hauptkette verbunden sind. Übliche kationische Gruppen enthalten quartäre Stickstoff- oder Phosphoratome. Gruppen mit quartären Stickstoffatomen sind dabei bevorzugt. Die quartären Stickstoffatome können dabei sowohl 4 unterschiedliche oder z.T. gleiche Substituenten tragen, als auch Teil eines Ringsystems sein. Bevorzugte kationische Gruppen sind Ammonium- und Imidazoliniumgruppen.

Befinden sich die ionischen Gruppen in den Seitenketten, so sind die Polymeren aus Verbindungen aufgebaut, die neben mindestens einer kationischen Gruppe mindestens eine polymerisierbare Gruppe enthalten und frei sind von anionischen Gruppen.

Die polymerisierbare Gruppe ist bevorzugt eine Vinylgruppe. Es sind jedoch auch kationische Polymerisate verwendbar, bei denen die Polymerhauptkette beispielsweise aus Glykosiden aufgebaut ist oder Proteincharakter hat.

Erfindungsgemäß ebenfalls bevorzugt sind kationische Copolymere, die neben den kationischen Monomeren noch mindestens ein nichtionisches Monomer enthalten. Geeignete nichtionische Monomere sind beispielsweise Vinylpyrrolidon, Vinylacetat, Acrylamid, Methacrylamid, Methylacrylat, Ethylacrylat, Methylmethacrylat und Ethylmethacrylat. Vinylpyrrolidon ist ein besonders bevorzugtes nichtionisches Monomer.

Eine Reihe von für die Haarpflege geeigneten kationischen Polymeren sind dem Fachmann bekannt und als Handelsprodukte erhältlich.

Beispiele für solche Polymeren sind:
- quaternierte Cellulose-Derivate, wie sie unter den Bezeichnungen Celquat^{R} und Polymer JR^{R} im Handel erhältlich sind. Die Verbindungen Celquat H 100, Celquat L 200 und Polymer JR^{R}400 sind bevorzugte quaternierte Cellulose-Derivate.
- quaternierte Guar-Derivate, wie sie unter den Bezeichnungen Cosmedia Guar^{R} und Jaguar^{R} im Handel erhältlich sind. Bevorzugte Guar-Derivate sind beispielsweise Cosmedia Guar^{R} C-261 und Jaguar^{R} C 13-S.
- Copolymere des Vinylpyrrolidons mit quaternierten Derivaten des Dialkylaminoacrylats- und -methacrylats, wie beispielsweise mit Diethylsulfat quaternierte Vinylpyrrolidon-Dimethylaminomethacrylat-Copolymere sowie das Vinylpyrrolidon-Methacrylamidopropyltrimethylammoniumchlorid-Copolymere. Solche Verbindungen sind unter den Bezeichnungen Gafquat^{R}734, Gafquat^{R}755 bzw. Gafquat^{R} HS100 im Handel erhältlich.
- Copolymerisate des Vinylpyrrolidons mit Vinylimidazoliummethochlorid, wie sie unter der Bezeichnung Luviquat^{R} angeboten werden.
- Polymere Dimethyldiallylammoniumsalze und deren Copolymere mit Estern und Amiden von Acrylsäure und Methacrylsäure. Die unter den Bezeichnungen Merquat^{R}100 (Poly(dimethyldiallylammoniumchlorid)) und Merquat^{R}550 (Dimethyldiallylammoniumchlorid-Acrylamid-Copolymer) im Handel erhältlichen Produkte sind Beispiele für solche kationischen Polymere.
- Kationisch derivatisierte Silikonöle, wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).
- Kationisch derivatisierte Proteinhydrolysate, die beispielsweise durch Umsetzung von alkalisch, sauer oder enzymatisch hydrolysierten Proteinen mit Glycidyltrialkylammoniumsalzen oder 3-Halo-2-hydroxypropyltrialkylammoniumsalzen erhalten werden können, sind im Sinne dieser Erfindung ebenfalls kationische Polymere. Die Proteine, die als Ausgangstoffe für die Proteinhydrolysate dienen, können sowohl tierischer als auch pflanzlicher Herkunft sein. Übliche Ausgangsstoffe sind beispielsweise Keratin, Kollagen, Elastin, Sojaprotein, Milchprotein, Weizenprotein, Seidenprotein und Mandelprotein. Durch die Hydrolyse entstehen Stoffmischungen mit Molmassen im Bereich von ca. 100 bis ca. 50 000 Dalton. Übliche mittlere Molmassen liegen in einem Bereich von etwa 500 bis etwa 5000 Dalton. Nähere Einzelheiten über kationische Derivatisierung können u.a. der japanischen Patentanmeldung 77/73485 (Chemical Abstracts Referat 90:174508v) entnommen werden.

Vorteilhafterweise enthalten die kationisch derivatisierten Proteinhydrolysate eine oder zwei lange Alkylketten mit 8 bis 22 C-Atomen und entsprechend zwei oder eine kurze Alkylkette mit 1 bis 4 C-Atomen. Verbindungen, die eine lange Alkylkette enthalten, sind bevorzugt. Bevorzugte Proteinderivate sind Substanzen der Formel (II), 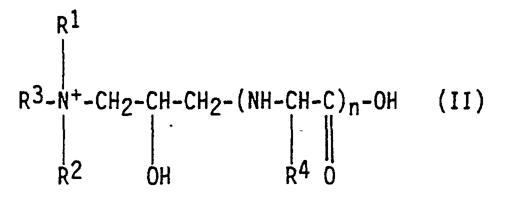 in der R⁴ für die Seitenketten der Aminosäuren des Proteins, R¹ und R² unabhängig voneinander für Alkylketten mit 1 bis 4 C-Atomen und R³ für eine Alkylkette mit 8 bis 22 C-Atomen steht.

Ein auf dem Markt erhältliches Produkt ist Lamequat^{R}L (Chemische Fabrik Grünau).
- Polymere Kondensationsharze von Polyolen und Polyaminen, wie beispielsweise Polyglykol-Polyamin-Kondensationsharze, die unter der CTFA-Bezeichnung PEG-15 Cocopolyamine bekannt sind. Im Handel ist beispielsweise das Produkt Polyquart^{R}H 81 (Henkel) erhältlich.

Unter "amphoteren Polymeren" sollen im Sinne der Erfindung Polymere verstanden werden, die im Molekül sowohl freie Aminogruppen als auch freie -COOH- oder -SO₃H-Gruppen enthalten und zur Ausbildung innerer Salze befähigt sind. Unter "zwitterionischen Polymeren" werden solche Polymeren verstanden, die im Molekül quartäre Ammoniumgruppen und -COO⁻- oder -SO₃⁻-Gruppen enthalten.

Beispiele für erfindungsgemäß einsetzbare amphotere Polymere sind die unter den Bezeichnungen Amphomer^{R} und Amphomer^{R} LV-71 erhältlichen Acrylharze, die Copolymere aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellen.

Geeignete zwitterionische Polymere sind beispielsweise die in den deutschen Patentanmeldungen DE 39 29 973, DE 21 50 557, DE 28 17 369 und DE 37 08 451 offenbarten Polymerisate. Acrylamidopropyltrimethylammoniumchlorid/Acrylsäure- bzw. -Methacrylsäure-Copolymerisate und deren Alkaliund Ammoniumsalze sind besonders bevorzugte zwitterionische Polymere. Weiterhin geeignete zwitterionische Polymere sind Methacroylethylbetain/Methacrylat-Copolymere, die unter der Bezeichnung Amersette^{R} (AMERCHOL) im Handel erhältlich sind. Ebenfalls bevorzugte zwitterionische Polymerisate sind Polysiloxan-Polyorganobetain-Copolymere sowie zwitterionische Celluloseether gemäß DE-OS-38 33 658.

Wie bereits oben erwähnt, wird der Fachmann hinsichtlich der kationischen und zwitterionischen Polymeren bevorzugt solche Verbindungen einsetzen, die keine Halogenidionen enthalten. Ganz besonders bevorzugt kationische Polymere sind daher die Handelsprodukte Gafquat^{R}755 und Abil^{R}-Quat 3270 und 3272.

Allerdings ist das Leistungsvermögen einiger halogenidhaltiger Polymerer so groß, daß bereits solche Mengen zur Erzielung des gewünschten Effekts ausreichen, die unterhalb der oben genannten Höchstgrenze liegen. In diesen Fällen wird der Fachmann anhand der Gesamtrezeptur entscheiden, ob er die halogenidhaltigen Polymeren einsetzt.

Weitere Bestandteile der erfindungsgemäßen Zubereitungen können beispielsweise sein:
- anionische Tenside wie beispielsweise Seifen, Alkylsulfate und Alkylpolyglykolethersulfate, Salze von Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist, Acylsarcoside, Acyltauride, Acylisethionate, Sulfobernsteinsäuremono- und dialkylester, lineare Alkansulfonate, lineare Alpha-Olefinsulfonate, alpha-Sulfofettsäuremethylester und Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.
- nichtionische Tenside wie beispielsweise Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe, C₁₂-C₂₂-Fettsäuremono- und -diester von Anlagerungsprodukten von 1 bis 30 Mol Ethylenoxid an Glycerin, C₈-C₂₂-Alkylmono- und -oligoglycoside und deren ethoxylierte Analoga sowie Anlagerungsprodukte von 5 bis 60 Mol Ethylenoxid an Rizinusöl und gehärtetes Rizinusöl.
- Proteinhydrolysate wie beispielsweise Kollagen-Hydrolysate, Elastin-Hydrolysate, Keratin-Hydrolysate, Hydrolysate von Weizenproteinen, Milchproteinen, Eiweißproteinen, Seidenproteinen, Mandelproteinen, Sojaeiweiß sowie Proteinen aus Tierhäuten, Kollagenhydrolysat-Kondensate mit organischen Säuren, wie beispielsweise Ölsäure, Myristinsäure, Undecylensäure, Kokosfettsäure und Abietinsäure, und deren Salze, Elastinhydrolysat-Kondensate mit Fettsäuren.
- Verdickungsmittel wie Agar-Agar, Guar-Gum, Alginate, Xanthan-Gum und Cellulose-Derivate wie Hydroxyethyl- und Methylhydroxypropyl-Cellulose,
- Strukturanten wie Glucose und Maleinsäure,
- Anionische und nichtionische Polymere wie beispielsweise Vinylacetat/Crotonsäure-Copolymere, Vinylpyrrolidon/ Vinylacrylat-Copolymere, Vinylacetat/Butylmaleat/Isobornylacrylat-Copolymere, Methylvinylether/Maleinsäureanhydrid-Copolymere und deren Ester, unvernetzte und mit Polyolen vernetzte Polyacrylsäuren, Polyvinylpyrrolidon, Vinylpyrrolidon/Vinylacetat-Copolymere.
- Lösungsvermittler, wie Ethanol, Isopropanol, Glycerin und Diethylenglykol,
- Substanzen zur Einstellung des pH-Wertes wie Natronlauge, Ammoniak, Ammoniumcarbonat, Ammoniumcarbamat und Citronensäure/Natriumcitrat-Puffer,
- Wirkstoffe wie Panthenol, Allantoin, Pyrrolidoncarbonsäuren, Pflanzenextrakte und Vitamine,
- Lichtschutzmittel,
- Komplexbildner wie EDTA, NTA und Phosphonsäuren,
- Stabilisatoren für Wasserstoffperoxid wie beispielsweise Phosphorsäure, Phosphonsäuren, Benzoesäure, Gluconsäure, Dipicolinsäure und EDTA,
- direktziehende Farbstoffe,
- Parfümöle, Dimethylisosorbid und Cyclodextrine,
- Treibmittel wie Propan-Butan-Gemische, N₂O, Dimethylether und Luft

Gegenstand der Erfindung ist auch ein Verfahren zur Behandlung von Haaren unter Verwendung eines Mittels gemäß einem der Ansprüche 1 bis 8.

### Beispiele

Alle Angaben sind Gewichtsteile.

| 1) Schaumaerosolfixierung | |
|---|---|
| Wasserstoffperoxid, 50 %ig | 5,0 |
| Eumulgin^{R}HRE 40¹ | 4,0 |
| Texapon^{R}NSO² | 6,0 |
| Gafquat^{R}755³ | 1,0 |
| Citronensäure | 0,3 |
| Propan/Butan | 3,5 |
| Parfümöl, Stabilisatoren | q.s. |
| Wasser | ad 100 |

| | |
|---|---|
| ¹ hydriertes Rizinusöl + 40 Ethylenoxid (CTFA-Bezeichnung: PEG-40-Hydrogenated-Castor-Oil) (HENKEL) | |
| ² Natriumlaurylethersulfat (CTFA-Bezeichnung: Sodium Laureth Sulfate; 28 % Aktivsubstanz) (HENKEL) | |
| ³ Dimethylaminoethylmethacrylat-Vinylpyrrolidon-Copolymer, quaterniert mit Diethylsulfat (19 % Aktivsubstanz in Wasser; CTFA-Bezeichnung: Polyquaternium-11) (GAF) | |

| 2) Schaumaerosolfixierung | |
|---|---|
| Wasserstoffperoxid, 50 %ig | 4,6 |
| Texapon^{R}NSO | 7,0 |
| Plantaren^{R} 2000⁴ | 1,5 |
| Tego^{R} Betain L 5351⁵ | 3,0 |
| Gelita^{R}Sol CS 50⁶ | 0,3 |
| Citronensäure | 0,4 |
| Propan/Butan | 4,0 |
| Parfümöl, Stabilisatoren | q.s. |
| Wasser | ad 100 |

| | |
|---|---|
| ⁴ C₈₋₁₆-Alkyl-1,4-polyglucosid (ca. 53 % Aktivsubstanz; CTFA-Bezeichnung: Decyl Polyglucose) (HENKEL) | |
| ⁵ Alkylamidobetain (33 % Aktivsubstanz in Wasser; CTFA-Bezeichnung: Cocoamidopropyl Betaine) (GOLDSCHMIDT) | |
| ⁶ Kollagenpartialhydrolysat (ca. 48,5 % in Wasser; CTFA-Bezeichnung: Hydrolyzed Collagen) (DEUTSCHE GELATINE FABRIKEN STOESS AG) | |

| 3) Schaumaerosolfixierung | |
|---|---|
| Wasserstoffperoxid, 50 %ig | 4,4 |
| Eumulgin^{R}HRE 60⁷ | 4,5 |
| Texapon^{R}NSO | 5,5 |
| Dehyquart^{R}SP⁸ | 3,0 |
| Protaflor Silk Q⁹ | 0,3 |
| Acrylamidopropylmethylammoniumchlorid/Acrylsäure(70:30)-Copolymeres, mit Natronlauge neutralisiert | 0,2 |
| Na₂HPO₄ | 0,3 |
| Citronensäure | 0,3 |
| Propan/Butan | 3,8 |
| Parfümöl, Stabilisatoren | q.s. |
| Wasser | ad 100 |

| | |
|---|---|
| ⁷ hydriertes Rizinusöl + 60 Ethylenoxid (CTFA-Bezeichnung: PEG-60-Hydrogenated-Castor-Oil) (HENKEL) | |
| ⁸ Oxyethylalkylammoniumphosphat (50 % Aktivsubstanz; CTFA-Bezeichnung: Quaternium-52) (HENKEL) | |
| ⁹ quaterniertes Seidenprotein-Hydrolysat und Amphotensid (35 % Aktivsubstanz; CTFA-Bezeichnung: Quaternized Silk Protein Hydrolysate (and) Cocoamphodipropionate) (CRODA) | |

| 4) Schaumaerosolfixierung | |
|---|---|
| Wasserstoffperoxid, 50 %ig | 6,0 |
| Eumulgade^{R}F spezial¹⁰ | 2,0 |
| Texapon^{R}NSO | 6,0 |
| Merquat^{R}550¹¹ | 0,5 |
| Na₂HPO₄ | 0,3 |
| Citronensäure | 0,3 |
| Propan/Butan | 3,8 |
| Parfümöl, Stabilisatoren | q.s. |
| Wasser | ad 100 |

| | |
|---|---|
| ¹⁰ Cetylstearylalkohol-Emulgator-Gemisch (CTFA-Bezeichnung: Cetearyl Alcohol (and) PEG-40 Castor Oil) (HENKEL) | |
| ¹¹ Dimethyldiallylammoniumchlorid-Acrylamid-Copolymer (8 % Aktivsubstanz; CTFA-Bezeichnung: Polyquaternium 7) (MOBIL OIL) | |

## Patentansprüche

1. Abgabevorrichtung zum Ausbringen eines darin konfektionierten, korrosive Komponenten enthaltenden Mittels, das einem Gehalt an Halogeniden von weniger als 0,5 Gew.-%, berechnet als Natriumchlorid, und das 0,1-50 Gew.-% an korrosiven Bestandteilen, jeweils bezogen auf das gesamte Mittel, aufweist, **dadurch gekennzeichnet, daß** die mit dem Mittel in Kontakt kommenden Teile des Ventils der Abgabevorrichtung aus Edelstahl bestehen oder mit einem Polyolefin beschichtet sind.

2. Abgabevorrichtung nach Anspruch 1, **dadurch gekennzeichnet, daß** die korrosive Komponente ausgewählt ist aus Phosphonsäuren, Alkalimetallhydroxiden und Wasserstoffperoxid.

3. Abgabevorrichtung nach einem der Ansprüche 1 oder 2, **dadurch gekennzeichnet, daß** die Halogenide vollständig durch andere Rohstoffe, in denen sie herstellungsbedingt enthalten sind, eingebracht werden.

4. Abgabevorrichtung nach einem der Ansprüche 1 bis 3, **dadurch gekennzeichnet, daß** das darin konfektionierte Mittel zusätzlich kationische, zwitterionische oder amphotere Tenside enthält.

5. Abgabevorrichtung nach einem der Ansprüche 1 bis 4, **dadurch gekennzeichnet, daß** das darin konfektionierte Mittel zusätzlich kationische, zwitterionische oder amphotere Polymere enthält.

6. Abgabevorrichtung nach einem der Ansprüche 1 bis 5, **dadurch gekennzeichnet, daß** das darin konfektionierte Mittel ein Treibmittel, ausgewählt aus Kohlenwasserstoffen, Dimethylether, Stickstoff und Luft enthält.

7. Abgabevorrichtung nach einem der Ansprüche 1 bis 6, **dadurch gekennzeichnet, daß** das darin konfektionierte Mittel ein Mittel zur Behandlung von Haaren, insbesondere ein Mittel im Rahmen einer Dauerwellbehandlung oder einer oxidativen Färbebehandlung, ist.

8. Abgabevorrichtung nach einem der Ansprüche 1 bis 7, **dadurch gekennzeichnet, daß** es sich um eine Spraydose oder Pumpspray-Dose handelt.

9. Abgabevorrichtung nach Anspruch 8, **dadurch gekennzeichnet, daß** der aus Edelstahl bestehende oder mit Polyolefin beschichtete Teil des Ventils der Ventilteller oder die Ventilfeder ist.

10. Abgabevorrichtung nach Anspruch 9, **dadurch gekennzeichnet, daß** der Ventilteller aus Edelstahl besteht.

11. Abgabevorrichtung nach einem der Ansprüche 8 bis 10, **dadurch gekennzeichnet, daß** sich das darin konfektionierte Mittel in einem Kunststoffinnenbeutel befindet.

12. Abgabevorrichtung nach Anspruch 11, **dadurch gekennzeichnet, daß** der Innenbeutel aus einem Polyolefm, insbesondere Polyethylen, besteht.

## Claims

1. A dispenser for dispensing a formulation containing corrosive components which is prepared in the dispenser and which contains less than 0.5% by weight of halides, expressed as sodium chloride, and 0.1 to 50% by weight of corrosive constituents, based on the formulation as a whole, **characterized in that** those parts of the valve of the dispenser which come into contact with the formulation consist of stainless steel or are coated with a polyolefin.

2. A dispenser as claimed in claim 1, **characterized in that** the corrosive component is selected from phosphonic acids, alkali metal hydroxides and hydrogen peroxide.

3. A dispenser as claimed in claim 1 or 2, **characterized in that** the halides are entirely introduced by other raw materials in which they are present from the production process.

4. A dispenser as claimed in any of claims 1 to 3, **characterized in that** the formulation prepared therein additionally contains cationic, zwitterionic or amphoteric surfactants.

5. A dispenser as claimed in any of claims 1 to 4, **characterized in that** the formulation prepared therein additionally contains cationic, zwitterionic or amphoteric polymers.

6. A dispenser as claimed in any of claims 1 to 5, **characterized in that** the formulation prepared therein contains a propellent selected from hydrocarbons, dimethyl ether, nitrogen and air.

7. A dispenser as claimed in any of claims 1 to 6, **characterized in that** the formulation prepared therein is a formulation for the treatment of hair, more particularly a formulation for use in permanent waving or oxidative colouring treatments.

8. A dispenser as claimed in any of claims 1 to 7, **characterized in that** it is a spray can or pump spray can.

9. A dispenser as claimed in claim 8, **characterized in that** the valve component consisting of stainless steel or coated with polyolefin is the valve disk or the valve spring.

10. A dispenser as claimed in claim 9, **characterized in that** the valve disk consists of stainless steel.

11. A dispenser as claimed in any of claims 8 to 10, **characterized in that** the formulation is accommodated in an inner plastic bag.

12. A dispenser as claimed in claim 11, **characterized in that** the inner bag consists of a polyolefin, more particularly polyethylene.

## Revendications

1. Dispositif distributeur pour l'application d'un produit comportant des composants corrosifs contenu dans celui-ci et qui présente une concentration en halogénures, calculée comme chlorure de sodium, inférieure à 0,5 % en poids et de 0,1 à 50 % en poids de composants corrosifs (dans chaque cas par rapport à la totalité du produit), qui est **caractérisé en ce que** les parties de la soupape du dispositif distributeur entrant en contact avec le produit sont en acier spécial ou sont revêtues d'une polyoléfine.

2. Dispositif distributeur selon la revendication 1, **caractérisé en ce que** le composant corrosif est sélectionné parmi les acides phosphoniques, les hydroxydes de métaux alcalins et l'eau oxygénée.

3. Dispositif distributeur selon une des revendications 1 ou 2, **caractérisé en ce que** les halogénures sont incorporés complètement via d'autres matières premières dans lesquelles elles sont contenues de par leur fabrication.

4. Dispositif distributeur selon une des revendications 1 à 3, **caractérisé en ce que** le produit conditionné dans celui-ci contient en plus des tensioactifs cationiques, zwitterioniques ou amphotères.

5. Dispositif distributeur selon une des revendications 1 à 4, **caractérisé en ce que** le produit conditionné dans celui-ci contient en plus des polymères cationiques, zwitterioniques ou amphotères.

6. Dispositif distributeur selon une des revendications 1 à 5, **caractérisé en ce que** le produit conditionné dans celui-ci contient un agent propulseur sélectionné parmi les hydrocarbures, les éthers diméthyliques, l'azote et l'air.

7. Dispositif distributeur selon une des revendications 1 à 6, **caractérisé en ce que** le produit conditionné dans celui-ci contient un agent pour traiter les cheveux, en particulier un agent utilisé dans le cadre d'une permanente ou d'une coloration par oxydation.

8. Dispositif distributeur selon une des revendications 1 à 7, **caractérisé en ce qu'**il s'agit d'un spray ou d'un spray à pompe.

9. Dispositif distributeur selon la revendication 8, **caractérisé en ce que** la partie de la soupape en acier spécial ou revêtue de polyoléfine est le siège ou le ressort de la soupape.

10. Dispositif distributeur selon la revendication 9, **caractérisé en ce que** le siège de soupape est en acier spécial.

11. Dispositif distributeur selon une des revendications 8 à 10, **caractérisé en ce que** le produit conditionné dans celui-ci se trouve dans un sachet interne en matière plastique.

12. Dispositif distributeur selon la revendication 11, **caractérisé en ce que** le sachet interne est constitué d'une polyoléfine, en particulier de polyéthylène.
